# EUROPEAN PATENT APPLICATION

(11) **EP 1 180 364 A1**
(43) Date of publication of application: **20.02.2002**
(21) Application number: 00202858.7
(22) Date of filing: 15.08.2000
(51) Int. Cl.: A61K 9/14, A61K 47/28

(54) **Steroid hormones as transfer agents**

(71) Applicant: Octagene GmbH, 80639 München (DE)
(72) Inventor: Hauser, Charlotte, Dr., 80639 München (DE)
(74) Representative: Helbing, Jörg, Dr. Dipl.-Chem.

(57) **Abstract**

The invention relates to compositions containing at least one stereoid hormone and a non-steroidal pharmaceutically active compound which compositions are capable of transferring the pharmaceutically active compounds into cells.

## Description

### Object of the Invention

The invention relates to compositions containing at least one steroid hormone and a non-steroidal pharmaceutically active compound which compositions are capable of transferring the pharmaceutically active compounds into cells.

### Summary of the Related Art

The transfer of pharmaceutically active compounds into cells is a goal for pharmaceutical research. Not only for gene therapy which involves the transfer of recombinant genes or transgenes into somatic cells to replace proteins with a genetic defect or to interfere with the pathological process of an illness, but also for transferring non-DNA compounds a safe and easy system is still wanted. Currently the transfer of non-DNA compounds into cells is achieved via oral, nasal or mucosal administration, subcutaneous or i.v. application, or the like. However, in these methods the compounds have to be adopted to pass membrane barriers which consequently imposes severe limitations on the structures of the compounds to be administered.

With regard to gene therapeutic methods it is referred to the methods summarized in PCT/EP00/01368 (filed March 3, 2000).

On the other hand, the classical model of the action of hormones is based on the concept of binding interaction of the hormone to an intracellular receptor, located in the cytoplasm or the nucleus (Evans, *R., Science,* Vol. 240, 889, 1988). These intracellular receptors remain latent until exposed to their target hormone. When so exposed, the hormone receptor changes its conformation after the hormone is bound and translocates in the activated form into the cell nucleus where it binds as a dimer to hormone responsive elements in the promoter region of hormone-regulated genes (Beato, M., *Cell,* Vol. 56, 335, 1989; O'Malley, B., et al., *Biol. Reprod.,* Vol. 46, 163, 1992). The hormone responsive elements are enhancer elements usually located in the 5' flanking region of the specific hormone-induced gene, i.e., are functionally linked to the specific hormone induced gene. DNA constructs comprising a hormone responsive element and a nucleic acid sequence encoding a protein of interest are disclosed in U.S. Pat. Nos. 5,688,677 and 5,580,722 and are taught to be suitable for expression of the protein of interest.

An example of such intracellular receptors is the steroid receptor. Steroid receptors belong to a superfamily of ligand-dependent transcription factors characterized by a unique molecular structure. The centrally located highly conserved DNA-binding domain defines this superfamily. The second important and relatively invariant region is the COOH-terminal ligand-binding domain. An example of such a receptor is the progesterone receptor mediated by the steroid progesterone. At the progesterone receptor, progesterone acts as a natural agonist whereas it displays potent antimineralocorticoid properties both at the molecular and the systemic level. Besides classical effects on the uterus, antiepileptic, anxiolytic, hypnotic and anesthetic properties have been attributed to progesterone according to numerous studies. Specifically, U.S. Patents Nos. 4,196,188 and 5,140,021 disclose pharmaceutical compositions comprising finely ground progesterone particles having a particle size of less than 15 and 10 µm, respectively and being suspended in an oil vehicle (so-called "micronized progesterone"). The compositions are suitable as contraceptive and for the treatment of premenstrual syndrome.

Recently it was found that steroid hormones, e.g., such as the micronized progesterone, are very effective mediators for the transfer of nucleic acid constructs through the cell membranes into a cell (PCT/EP00/01368). It was now found that such steroid hormones are also effective mediators of (non-nucleic acid) pharmaceutically active compounds through the cell membranes into a cell.

### Brief Description of the Invention

The present invention thus provides
(1) a pharmaceutical composition comprising at least one steroid hormone and a non-steroidal pharmaceutically active compound;
(2) a preferred embodiment of (1) above, wherein the steroid hormone is a natural micronized steroid hormone;
(3) use of steroid hormone for preparing an agent for transferring a non-steroidal pharmaceutically active compound into cells; and
(4) a method for transferring a non-steroidal pharmaceutically active compound to an organism or to a cellular system, which method comprises administering the active compound to the organism or to the cellular system in admixture with a steroid hormone.

### Detailed Description of the Invention

### 1. Definitions

"Non-steroidal pharmaceutically active compounds" (hereinafter also shortly referred to as "pharmaceutical compounds") refers to pharmaceutically active compounds which are not a steroid hormone such as low molecular pharmaceutically active compounds and polymeric structures, except polymeric structures essentially comprised of nucleic acids such as nucleic acid constructs.

In the above "nucleic acid" means DNA, cDNA, mRNA, tRNA, rRNA. The nucleic acid may be linear or circular, double-stranded or single-stranded, and "nucleic acid construct" refers to a composite of nucleic acid elements in relation to one another. The nucleic acid elements of the construct may be incorporated into a vector in such an orientation that a desired gene may be transcribed, and if desired, a desired protein may be expressed. Polymeric structures are essentially comprised of amino acid residues (e.g., proteinacious structures), polyhydroxy compounds (such as carbohydrates and glycoproteins) are, however, included in the "non-structural pharmaceutically active compounds".

"Organism" refers to a multicellular living entity including vertebrates such as mammals (especially humans, cattle, rodents, dogs) and invertebrates.

"Cellular system" includes cell cultures, e.g., primary cell cultures (especially those suitable for reimplantation), stem cells, blood cells, tissue samples and whole organs and immortalized cell cultures.

"Therapeutically effective dose" within the ambit of the present invention refers to a dose effective for treatment or prophylaxis, or a dose that is predictable to be effective for treatment or prophylaxis by extrapolating from *in vitro* or *in vivo* data. The determination of a therapeutically effective dose is within the purview of one skilled in the art.

### 2. Detailed Description and Examples

As stated above, an object of the present invention is to provide a new and improved delivery system.

The steroid hormone in the composition of embodiment(s) (1) and (2) and in the medicament of the embodiment (3) include synthetic and natural steroid hormones, such as estrogen, testosterone, glucocorticoid, androgen, thyroid hormone, and progesterone or derivatives thereof. These are widely available. Progesterone is most preferred. For example, natural micronized progesterone is the preferred progesterone from which has been marketed in France since 1980 under the trademark of UTROGESTAN® and is still available in Germany under the trademark UTROGEST® . Its properties are similar to the endogenous progesterone, in particular, it has antiestrogen, gestagen, slightly antiandrogen and antimineralocorticoid properties. The natural micronized progesterone in said marketed products is dispersed in a matrix as described herein below.

The above micronized progesterone has advantages that make it a suitable carrier for genes or nucleic acid constructs to target cells. Specifically, the synergistic effect of the double process of micronization and suspension a lipophilic matrix (see below), especially in long-chain fatty acids residues of an oil results in increasing progesterone absorption. It has been demonstrated that after oral administration of 100 mg of UTROGESTAN® , peak plasma progesterone levels were obtained after 1-4 hours in most cases (Padwick, M. L., et al., *Fertil. Steril.,* Vol. 46, 402, 1986). Later on, the levels declined substantially, although they were still elevated at 12 hours. Even at 84 hours the levels were slightly higher than baseline. A U.S. kinetic study confirmed earlier work demonstrating the bioavailability of oral micronized progesterone. They showed a peak effect at 2 hours followed by rapid decrease in plasma progesterone level (Simon, J. A., et al., *Fertil.,. Steril.,* Vol., 60, 26, 1993).

A further advantage of using progesterone as a carrier is the low level of disadvantageous side effects. Orally administered progesterone adversely affects neither plasma lipids (Jensen, J. et al., Am. J. Obstet. Gynecol., Vol. 156, 66, 1987) nor carbohydrate metabolism (Mosnier-Pudar, H. et al., Arch. Mal. Coeur, Vol 84, 1111, 1991). Further, progesterone does not affect liver enzymes (ASAT, ALAT, AFOS), sex-hormone binding-globulin (SHBG) synthesis or HDL-cholesterol levels at daily doses of 200 mg and 300 mg. Although the plasma levels of deoxycorticosterone may increase substantially during UTROGESTAN® treatment, there are strong indications that the mineralocorticoid effects of this progesterone metabolite are completely counteracted by the anti-mineralocorticoid effects of progesterone itself. This is apparent from a comparative study (Corvol, P., et al., In: Progesterone and progestins. Raven Press, New York, 179, 1983) in which oral UTROGESTAN® was capable of antagonizing the mineralocorticoid effects of 9-α-fluorohydrocortisone.

In the composition of embodiment (1) and (2) and in the medicament of embodiment (3) of the invention the molar ratio of hormone to pharmaceutical compound is at least 50:1, preferably at least 1000:1, more preferably at least 10000:1.

The skilled artisan will appreciate that the composition of embodiments (1) and (2) and the medicament of embodiment (3) may contain other components capable of assisting in introducing the nucleic acid into a cell for the purpose of gene therapy (e.g., the matrix compounds mentioned hereinbefore). Specifically, the composition and the medicament, especially the hormone component thereof, may contain the following matrix compounds: glucose and related compounds (such as D-sorbitol, D-mannitol); solubilizing adjuvants (such as alcohols, e.g., ethanol); polyhydric compounds such as glycerine, polyethylene glycol and polypropylene glycol; nonionic surface active compounds, ionic surface active compounds such as lecithin; oily compounds such as sesame oil, peanut oil soybean oil, corn oil, etc.; starches and their derivatives such as cyclodextrines and hydroxyalkylated starches; stabilizers such as human serum albumin, preservatives such as benzyl alcohol and phenol; and the like. The preferred matrix contains β-cyclodextrine, glycerine, lecithin and/or corn oil. In case of micronized steroid hormones such as micronized progesterone the micronized particles are suspended in an oil vehicle, preferably an oil vehicle which is high in glycerides of polyunsaturated fatty acids such as corn oil, sunflower oil and soybean oil (see U.S. Patent No. 5,140,021; the disclosure of which is incorporated herein by reference). Preferably, the oil:micronized steroid hormone ratio does not exceed 2.5 ml/g.

For example, the pharmaceutical composition of steroid hormone and pharmaceutical compound of the invention may be provided orally to humans or animals as a gelatin capsule. Progesterone therein (preferably in micronized form) could be present in a concentration of 50 to 1000 mg, preferably 200 -300 mg dissolved in a 35 % or 40 % β-cyclodextrin solution or in corn oil or gycerol with peanut oil together with lecithin.

Alternatively, when - due to the selection of appropriate matrix components - the pharmaceutical composition is in a pasty, gel-like form, it may be provided topically.

The composition of embodiment (1) of the invention can be prepared by admixing the pharmaceutical compound with the hormone. Preferably, an aqueous solution of pharmaceutical compound (if the compound is soluble in such aqueous systems) is added to the oily suspension containing the hormone at ambient temperature under stirring.

The dose of non-steroidal compound is dependent on the condition to be treated, the characteristics of the patient, and the result sought to be achieved. Determining dosage is within the realm of the skilled artisan.

The pharmaceutical composition and the medicament of the present invention may be administered orally, rectally, intravenously, intramuscularly, subcutaneously, topically or through mucosa (including buccal, nasal spray). Oral administration (of a micronized hormone dispersion) is preferred. Delivery may be systemic or directed at certain tissue.

Embodiments (3) and (4) of the invention pertain to the use of a steroid hormone for preparing an agent for transferring a non-steroidal pharmaceutically active compound to an organism and to a method for transferring a non steroidal pharmaceutically active compound to an organism or to a cellular system, which comprises administering the active compound to the organism or to a cell system. In a preferred embodiment the active compound is encapsulated in the steroid hormone. Suitable steroid hormones are enumerated above. The preferred steroid hormone in said embodiments of the invention is a natural micronized steroid hormone, in particular a natural micronized progesterone. In a preferred embodiment, the micronized hormone is solubilized/dispersed in a lipophilic matrix as set forth above. The dose and mode of administration is within the ambit of a person skilled in the art.

## Claims

1. A pharmaceutical composition comprising at least one micronized steroid hormone and a non-steroidal pharmaceutically active compound.

2. The composition of claim 1, wherein the steroid hormone is selected from estrogen, testosterone, glucocorticoid, androgen, thyroid hormone and progesterone and derivatives thereof, and preferably is progesterone.

3. The composition of claim 1 or 2, wherein the steroid hormone is a natural micronized steroid hormone.

4. the composition of claims 1 to 3, wherein the non-steroidal pharmaceutically active compound is a low molecular weight compound or a polymeric compound substantially comprised of amino acid residues.

5. The composition of claims 1 to 4 wherein the molar ratio of steroid hormone to non-steroidal pharmaceutically active compound is at least 50:1, preferably at least 1000:1.

6. The composition of claims 1 to 5 further comprising a lipophilic matrix.

7. The composition of claims 1 to 6, which is suitable for transferring pharmaceutically active compounds into cells.

8. Use of a steroid hormone for preparing a medicament for transferring a non-steroidal pharmaceutically active compound to an organism.

9. The use of claim 8, wherein the steroid hormone is a natural micronized steroid hormone, preferably natural micronized progesterone.

10. The use of claim 9, wherein the natural micronized steroid hormone is solubilized in a lipophilic matrix.

11. A method for transferring a non-steroidal pharmaceutically active compound to an organism or to a cellular system, which method comprises administering the active compound to the organism or to the celluloar system in admixture with a steroid hormone.

12. The method of claim 11 wherein the pharmaceutically active compound is encapsulated in the steroid hormone.
